# EUROPEAN PATENT APPLICATION

(11) **EP 4 837 538 A1**
(43) Date of publication of application: **11.11.2026**
(21) Application number: 24915221.6
(22) Date of filing: 30.12.2024
(51) Int. Cl.: A61F 2/24

(54) **ARTIFICIAL IMPLANT, INTERVENTIONAL SYSTEM, AND METHOD FOR DRIVING BLOCKING MEMBER ON ARTIFICIAL IMPLANT TO DEFORM**

(30) Priority: 02.01.2024 CN 202410006391; 02.01.2024 CN 202410002484
(71) Applicant: Venus MedTech (HangZhou), Inc., Hangzhou, Zhejiang 310052 (CN)
(72) Inventor: KUANG, Dajun, Hangzhou, Zhejiang 310052 (CN); LIU, Xianbao, Hangzhou, Zhejiang 310052 (CN); ZENG, Xiaotong, Hangzhou, Zhejiang 310052 (CN); XU, Shasha, Hangzhou, Zhejiang 310052 (CN); XIAO, Min, Hangzhou, Zhejiang 310052 (CN); HONG, Yang, Hangzhou, Zhejiang 310052 (CN); WANG, Chenghong, Hangzhou, Zhejiang 310052 (CN); NI, Yufei, Hangzhou, Zhejiang 310052 (CN); YANG, Yake, Hangzhou, Zhejiang 310052 (CN)
(74) Representative: KIPA AB
(86) International application number: PCT/CN2024/143638
(87) International publication number: WO 2025/146007

(57) **Abstract**

The present application discloses an artificial implant, an interventional system, and a method for driving deformation of a sealing assembly on an artificial implant. The artificial implant comprises: a stent; and a sealing assembly connected to the stent and arranged circumferentially around the stent, the sealing assembly being provided with a first guide structure, wherein when the stent is in an expanded state, at least a part of the sealing assembly protrudes outward from an outer periphery of the stent, and when the stent is in a compressed state, the sealing assembly folds at the first guide structure to accommodate deformation of the stent. The present application effectively reduces the incidence of paravalvular leakage through the provision of the sealing assembly. The first guide structure enables the sealing assembly to adapt to the deformation of the stent, effectively overcoming the drawbacks of access limitation and susceptibility to detachment and damage, thereby providing a sufficient structural basis for optimized design of artificial implants.

## Description

### TECHNICAL FIELD

The present application relates to the field of medical devices, and in particular, to an artificial implant, an interventional system, and a method for driving deformation of a sealing assembly on an artificial implant.

### BACKGROUND

Heart valve replacement surgery has been widely used clinically as an effective means for treating heart valve diseases, but the problem of paravalvular leakage (PVL) has not been properly solved in the industry. Paravalvular leakage is a non-structural valve dysfunction that often occurs in the early and late stages after prosthetic valve replacement. The main treatment methods for paravalvular leakage can be divided into surgical treatment and interventional treatment. Regardless of the method, there are problems such as increased complications, increased mortality, and a high incidence of recurrent paravalvular leakage.

Therefore, those skilled in the art have considered reducing the incidence of paravalvular leakage from the perspective of valve design. A common approach is to add a skirt design at the inflow end of the valve to seal the leakage site. Skirt materials are mainly biological skirts and polymer skirts. It is foreseeable that introducing a skirt will increase the delivery system profile and impose design restrictions on the prosthetic valve, and the skirt is prone to damage during delivery of the prosthetic valve, among other problems. This has seriously affected the development of this technical approach.

### TECHNICAL PROBLEM

In order to solve the above technical problems, the sealing assembly is further improved.

### TECHNICAL SOLUTION

The present application discloses an artificial implant, comprising:
a stent having a tubular structure, wherein the tubular structure is radially deformable and consequently has a corresponding compressed state and expanded state; and
a sealing assembly connected to the stent and arranged circumferentially around the stent, wherein when the stent is in the expanded state, at least a part of the sealing assembly protrudes outward from an outer periphery of the stent, and when the stent is in the compressed state, the sealing assembly is guided to fold to accommodate the deformation of the stent.

Several optional arrangements are also provided below. They are not intended to impose additional limitations on the above general solution, but rather to serve as further supplements or preferences. Provided there is no technical or logical contradiction, the optional arrangements may be combined with the above general solution individually, or multiple optional arrangements may be combined with each other.

Optionally, the deformation of the stent acts on a predetermined portion of the sealing assembly to guide the sealing assembly to fold, and during folding, at least a part of the sealing assembly moves radially inward of the stent.

Optionally, the deformation of the stent acts directly on the sealing assembly and/or indirectly acts on the sealing assembly.

Optionally, the sealing assembly has a first guide structure, and when the stent is in the compressed state, the sealing assembly folds at the first guide structure.

Optionally, the artificial implant further comprises:
an actuator connected to the stent, wherein during transition of the stent from the expanded state to the compressed state, the actuator acts radially inwardly on at least a part of the sealing assembly to guide folding of the sealing assembly.

Optionally, the stent has a grid structure, and during transition of the stent between states, an axial length of at least one grid of the grid structure changes correspondingly, and the actuator is configured to be driven by the change in axial length of the at least one grid.

Optionally, the actuator is driven by the stent to act radially inwardly on the sealing assembly.

Optionally, the actuator drives the deformation of the stent.

Optionally, the stent has a grid structure.

Optionally, the stent has an inflow side and an outflow side opposite each other in the axial direction under blood flow environment, the interior of the stent defines a blood flow channel, and the artificial implant further comprises leaflets connected to the stent for controlling the blood flow channel.

Optionally, wherein the leaflets include 2, 3, or 4 leaflets that cooperate with each other. Optionally, the leaflets are made of a biological material or a polymer material.

Optionally, the sealing assembly is located on the inflow side of the leaflets. Optionally, the first guide structure comprises at least one weakened portion, and when the stent is in the compressed state, the sealing assembly folds at the at least one weakened portion, thereby being radially inwardly collapsible toward the stent.

Optionally, the actuator is positioned in alignment with or circumferentially offset from the first guide structure.

Optionally, when the stent is in the expanded state, at least a part of the actuator extends through the weakened portion.

Optionally, the sealing assembly comprises a plurality of deformable sealing blocks arranged at intervals, and the weakened portion comprises a slit formed in one of the sealing blocks. Optionally, the slit has a constant width or a varying width.

Optionally, the width of the slit is 0~1 mm. Optionally, the sealing block is made of PU foam.

Optionally, the slit extends in a direction parallel to a generatrix of the stent. Optionally, in the radial direction of the stent, the depth of the slit is at least half of a thickness of the sealing block.

Optionally, in the radial direction of the stent, a depth of the slit penetrates through the sealing block, and the sealing block is maintained connected on both sides of the slit by a first component. Optionally, the first component is a reinforcing wire threaded through the sealing block.

Optionally, different parts of the sealing block have different thicknesses in the radial direction of the stent, and the slit intersects at least a part of the sealing block having the greatest thickness.

Optionally, the slit extends axially through the sealing block, or the slit is closed on at least one side.

Optionally, a reinforcing wire is arranged in the sealing block and is pulled on both sides of the slit.

Optionally, a threading path of the reinforcing wire avoids the stent or wraps around the stent.

Optionally, the reinforcing wire is fixed by tying or by an additional connector member.

Optionally, for the same slit, the reinforcing wire is a single thread reciprocally threaded through both sides of the slit.

Optionally, for the same slit, there are a plurality of reinforcing wires, and each reinforcing wire forms knots independently or in combination with other reinforcing wire

Optionally, the knots arranged at intervals along the extension direction of the slit.

Optionally, the weakened portion is a groove formed in the sealing block.

Optionally, an open side of the groove faces radially outward of the stent.

Optionally, a depth of the groove is at least half of a thickness of the sealing block.

Optionally, the groove penetrates through the sealing block, and the sealing block itself forms an annular structure.

Optionally, the groove is located at a geometric center of the sealing block.

Optionally, the first guide structure comprises at least one reinforced portion, and the reinforced portion drives other portions of the sealing assembly to fold and collapse radially inward of the stent.

Optionally, the reinforced portion is formed by increasing the strength of a partial region of the sealing assembly; or the reinforced portion is formed by a second component.

Optionally, the second component is a reinforcing wire threaded through the sealing assembly.

Optionally, in the radial direction of the stent, the depth of the reinforced portion is part or all of a thickness of the sealing assembly.

Optionally, the first guide structure comprises at least one weakened portion and a reinforced portion acting on the weakened portion, and the sealing assembly folds at the at least one weakened portion, thereby being radially inwardly collapsible toward the stent.

Optionally, the weakened portion forms a cavity region, and the reinforced portion is disposed around an inner circumferential surface of the cavity region; or the reinforced portion is located on an inner circumferential surface of the cavity region near the inner side of the stent.

Optionally, both sides of the weakened portion are connected by a reinforcing wire.

Optionally, the sealing assembly comprises a plurality of deformable sealing blocks, and the same sealing block has a plurality of first guide structures.

Optionally, the sealing assembly comprises a plurality of deformable sealing blocks, and all of the sealing blocks have the first guide structure, or only some of the sealing blocks have the first guide structure.

Optionally, the stent is self-expandable or balloon-expandable.

Optionally, the stent comprises an inner frame defining the blood flow channel and an outer framework connected to the inner frame and positioned around the outer periphery of the inner frame in the expanded state, the outer framework being made of a memory material suitable for self-expansion. The outer framework comprises arms arranged at intervals in the circumferential direction and/or an outer frame sleeved radially outside the inner frame.

Optionally, the stent is formed by cutting or braiding and has a plurality of grids, and during state switching, some or all of the grids deform adaptively. Optionally, the sealing assembly comprises deformable sealing blocks, and the sealing block is embedded in a correspondingly positioned grids.

Optionally, the sealing blocks are arranged in one or more groups along the axial direction of the stent, and sealing blocks in the same group are distributed circumferentially in the same ring of grids.

Optionally, the sealing blocks fill the entire grid, or only occupy a portion of the grid.

Optionally, the grid has a wide region with the largest span in the circumferential direction of the stent, and the first guide structure intersects the wide region. Optionally, the sealing block at least fills the wide region. Optionally, an extension direction of the first guide structure is perpendicular to an extension direction of the wide region.

Optionally, in the circumferential direction of the stent, the first guide structure is located at a middle portion of the sealing block, or is offset to one side.

Optionally, the first guide structures on all sealing blocks are offset in the same circumferential direction.

Optionally, the artificial implant further comprises:
an inner covering membrane distributed around an inner circumferential wall of the stent, the sealing assembly being fixed to an outer side of the inner covering membrane, and when the stent is in the expanded state, the sealing assembly protrudes outward of the stent through corresponding grids.

Optionally, the stent comprises an inner frame defining the blood flow channel and an outer framework connected to the inner frame and positioned around the outer periphery of the inner frame in the expanded state, and the inner covering membrane is distributed around an inner circumferential wall of the inner frame.

Optionally, the artificial implant further comprises: when the stent is in the compressed state, the folded portion of the sealing assembly together with the corresponding portion of the inner covering membrane forms an inward protrusion relative to the inner circumferential wall of the stent.

Optionally, in an axial view of the stent, at least 30% of the sealing assembly is located in the inward protrusion.

Optionally, the inner covering membrane is a PU film.

Optionally, the inner covering membrane is bonded to the sealing assembly or the sealing assembly is formed directly on a surface of the inner covering membrane.

Optionally, an outflow side of the inner covering membrane is spliced with leaflets and fixed to the stent.

Optionally, the inner covering membrane has a second guide structure.

Optionally, the second guide structure corresponds in position to the first guide structure in the circumferential direction of the stent.

Optionally, the artificial implant further comprises:
an outer covering membrane distributed around an outer circumferential wall of the stent, wherein part or all of the sealing assembly is wrapped by the outer covering membrane.

Optionally, the artificial implant further comprises an inner covering membrane distributed around an inner circumferential wall of the stent, wherein the outer covering membrane and the inner covering membrane are integrally formed.

Optionally, the outer covering membrane and the inner covering membrane together define a pocket structure having a radial gap, the sealing assembly is fixed to an outer surface of the inner covering membrane, and the entire sealing assembly is located within the pocket structure, or an outflow side of the sealing assembly is exposed outside the pocket structure.

Optionally, the inner covering membrane has a second guide structure, and the second guide structure is connected to the outer covering membrane.

Optionally, the outer covering membrane has a third guide structure.

Optionally, the third guide structure is connected to the inner covering membrane.

Optionally, the third guide structure is embedded in the first guide structure, and the embedded portion is fixedly connected to the sealing assembly and/or the inner covering membrane.

Optionally, the third guide structure is fixedly connected to the sealing assembly and/or the inner covering membrane at least at an outflow side edge portion of the outer covering membrane.

Optionally, the outer covering membrane has an axial width corresponding to at least one complete ring of grids, and the third guide structure is located at a midline portion of the corresponding grids.

Optionally, the inner covering membrane has a second guide structure, and the outer covering membrane has a third guide structure, each of the second and third guide structures independently being a low-strength band or a pre-formed crease.

Optionally, the low-strength band has a thinner thickness than adjacent portions or is made of a different material.

Optionally, the pre-formed crease is obtained by reciprocally folding and then heat-treating the material.

Optionally, the second guide structure and the third guide structure are respectively arranged at intervals in the circumferential direction along the stent.

Optionally, the number of the second guide structures and the third guide structures is equal to the number of grids in the axial direction of the stent, or is an integer multiple thereof.

The present application also discloses an artificial implant, comprising:
a stent having a tubular structure, wherein the tubular structure is radially deformable and consequently has a corresponding compressed state and expanded state;
an inner covering membrane distributed around an inner circumferential wall of the stent, the inner covering membrane being provided with a second guide structure, wherein when the stent is in the compressed state, the inner covering membrane folds at the second guide structure, thereby being radially inwardly collapsible toward the stent;
an outer covering membrane distributed around an outer circumferential wall of the stent, the outer covering membrane being provided with a third guide structure, wherein when the stent is in the compressed state, the outer covering membrane folds at the third guide structure, thereby being radially inwardly collapsible toward the stent.

The present application discloses an artificial implant, comprising:
a stent having a tubular structure and an interior defining a blood flow channel, wherein the tubular structure is radially deformable and consequently has a corresponding compressed state and expanded state;
leaflets connected to the stent for controlling the blood flow channel;
a sealing assembly connected to the stent and arranged circumferentially around the stent, wherein when the stent is in the expanded state, at least a part of the sealing assembly protrudes outward from an outer periphery of the stent; and
an actuator connected to the stent, wherein during transition of the stent from the expanded state to the compressed state, the actuator is driven by the stent to act radially inwardly on at least a part of the sealing assembly.

Optionally, the actuator acts on the sealing assembly in a line contact manner to radially inwardly compress the sealing assembly.

Optionally, the actuator is made of a polymer wire, a metal wire, or a hybrid wire of polymer and metal.

Optionally, the sealing assembly comprises a plurality of deformable sealing blocks arranged at intervals, and the actuator compresses one of the sealing blocks or simultaneously compresses the plurality of sealing blocks.

Optionally, the stent has a first connection point and a second connection point spaced apart in the axial direction, the actuator is a flexible member or a deformable structure, and at least one end of the actuator is connected to the first connection point, and at least the other end of the actuator is connected to the second connection point.

Optionally, the stent has a grid structure, and during a transition of the stent between states, an axial length of at least one grid of the grid structure changes correspondingly, and the actuator is configured to be driven by the change in axial length of the at least one grid.

Optionally, the actuator comprises at least one pulling wire, both ends of the pulling wire being constrained by two or more axially spaced portions of the stent, wherein in the expanded state the at least one pulling wire is slack to allow the sealing assembly to protrude outward, and in the compressed state the at least one pulling wire is taut to radially inwardly compress the sealing assembly.

Optionally, the at least one pulling wire of the actuator comprises a plurality of pulling wires arranged as follows:
the plurality of pulling wires extending in the axial direction of the stent; and/or at least one of the plurality of pulling wires extending in the circumferential direction of the stent; and/or at least one of the plurality of pulling wires extending simultaneously in the axial and circumferential directions of the stent; and/or a part of at least one of the plurality of pulling wires extending in the axial direction of the stent and another part extending in the circumferential direction of the stent.

Optionally, an end of the pulling wire is connected to the stent and/or to other pulling wires by one or more of tying, threading, wrapping, bonding, and welding.

Optionally, within at least one grid, one of the pulling wires extending in the axial direction of the stent and another one of the pulling wires extending in the circumferential direction of the stent cross each other, and the two pulling wires are positioned fixedly or movably relative to each other at a crossing point.

Optionally, one of the pulling wires has a threading ring for other pulling wires to pass through;
the threading ring is formed by looping the pulling wire itself; or the threading ring is independently provided and connected to the corresponding pulling wire.

Optionally, within a single grid, the pulling wire passes through a geometric center of the grid;
a plurality of pulling wires are provided and cross each other, and a crossing point is near the geometric center of the grid.

Optionally, each grid provided with the sealing assembly corresponds to at least one pulling wire, and the number of pulling wires in each grid provided with the sealing assembly is the same or different.

Optionally, the sealing assembly comprises a plurality of deformable sealing blocks arranged at intervals, and all of the sealing blocks are compressed by a single pulling wire.

Optionally, the stent is partially hollowed to form a grid structure, and a radial dimension of the pulling wire is smaller than or close to a radial dimension of a strut of the grid structure.

Optionally, the artificial implant further comprises:
an inner covering membrane distributed around an inner circumferential wall of the stent, the sealing assembly being fixed to an outer side of the inner covering membrane, and when the stent is in the expanded state, the sealing assembly protrudes outward of the stent through corresponding grids;
an outer covering membrane distributed around an outer circumferential wall of the stent, wherein part or all of the sealing assembly and/or the actuator is wrapped by the outer covering membrane. The present application also discloses an artificial implant, comprising:
a stent having a tubular structure and an interior defining a blood flow channel, wherein the tubular structure is radially deformable and consequently has a corresponding compressed state and expanded state;
leaflets connected to the stent for controlling the blood flow channel;
a sealing assembly connected to the stent and arranged circumferentially around the stent, wherein when the stent is in the expanded state, at least a part of the sealing assembly protrudes outward from an outer periphery of the stent;
a pulling wire, both ends of the pulling wire being constrained by two axially spaced portions of the stent, wherein in the expanded state the at least one pulling wire is slack to allow the sealing assembly to protrude outward, and in the compressed state the pulling wire is relatively taut to radially inwardly compress the sealing assembly.

The present application also discloses an artificial implant, comprising:
a stent having a tubular structure, wherein the tubular structure is radially deformable and consequently has a corresponding compressed state and expanded state;
a sealing assembly connected to the stent and arranged circumferentially around the stent, wherein when the stent is in the expanded state, at least a part of the sealing assembly protrudes outward from an outer periphery of the stent, the sealing assembly has a slit, and when the stent is in the compressed state, the sealing assembly is guided to fold at the slit to accommodate the deformation of the stent.

The present application also discloses a method for driving deformation of a sealing assembly on an artificial implant, comprising:
providing an artificial implant, the artificial implant at least comprising a stent and a sealing assembly, the sealing assembly being connected to the stent and arranged circumferentially around the stent, wherein when the stent is in the expanded state, at least a part of the sealing assembly protrudes outward from an outer periphery of the stent;
wherein a pulling wire is threaded through the stent, the pulling wire being slack to allow the sealing assembly to protrude outward, and when driving the sealing assembly to deform, the pulling wire is tightened so that it acts radially inwardly on at least a part of the sealing assembly to drive deformation of the sealing assembly.

Optionally, tightening the pulling wire is achieved entirely by deformation of the stent, or one end of the pulling wire is connected to the stent and the other end is a free end extending out of the stent, and tightening is performed by pulling the free end, or both ends of the pulling wire are free ends extending out of the stent, and tightening is performed by pulling one free end or both free ends.

The present application also discloses a method for driving deformation of a sealing assembly on an artificial implant, comprising:
providing an artificial implant, the artificial implant at least comprising a stent and a sealing assembly, the sealing assembly being connected to the stent and arranged circumferentially around the stent, wherein when the stent is in the expanded state, at least a part of the sealing assembly protrudes outward from an outer periphery of the stent; and
the sealing assembly has a slit, and when driving the sealing assembly to deform, the sealing assembly is compressed by deformation of the stent so that it is guided to fold at the slit.

The method for driving deformation of a sealing assembly on an artificial implant of the present application can be implemented either in vivo or during radial compression loading of the stent ex vivo, so as to load the artificial implant into a catheter assembly.The present application also discloses an interventional system, comprising:
an artificial implant according to any of the above technical solutions, being in a compressed state;
a catheter assembly, wherein at least one tube is inserted through the interior of the artificial implant and connected thereto; and at least one tube wraps around the outer periphery of the artificial implant; and
a control handle connected to and driving the catheter assembly.

In the interventional system, the artificial implant can be loaded into the catheter assembly using the method of the present application.

### BENEFICIAL EFFECTS

The technical solutions disclosed in the present application effectively reduce the incidence of paravalvular leakage through the provision of the sealing assembly. The guide structure or actuator can effectively drive the sealing assembly to adapt to the deformation of the stent, effectively overcoming the drawbacks of access limitation and susceptibility to detachment and damage, thereby providing a sufficient structural basis for optimized design of artificial implants.

### BRIEF DESCRIPTION OF THE DRAWINGS

FIG. 1 is a schematic view of an artificial implant according to an embodiment.
FIG. 2 is an enlarged schematic view of the sealing assembly of FIG. 1.
FIGS. 3 and 4 are schematic views of sealing assemblies according to different embodiments.
FIGS. 5 and 6 are schematic views of sealing assemblies in different states.
FIGS. 7a to 9d are schematic views of first guide structures according to different embodiments.
FIGS. 10a to 10f are schematic views of first guide structures according to different embodiments from another perspective.
FIG. 11 is a schematic view of an artificial implant according to another embodiment.
FIGS. 12 to 18 are schematic views of first guide structures according to different embodiments.
FIG. 19 is a schematic view of a stent according to an embodiment.
FIG. 20 is a schematic view of a grid of a stent according to an embodiment.
FIG. 21 is a schematic view showing cooperation between an inner covering membrane and a sealing block according to an embodiment.
FIG. 22 is a schematic view showing cooperation between an inner covering membrane and a sealing block according to another embodiment.
FIG. 23 is a schematic view of an artificial implant in axial view according to an embodiment.
FIGS. 24 and 25 are schematic views showing cooperation between a sealing block and a second guide structure according to different embodiments.
FIG. 26 is a schematic view of an inner covering membrane according to an embodiment.
FIG. 27 is a schematic view of an inner covering membrane and a sealing block in a compressed state.
FIG. 28 is a schematic view of an artificial implant according to yet another embodiment.
FIGS. 29 and 30 are schematic views of the artificial implant of FIG. 28 from different perspectives.
FIG. 31 is a schematic view showing cooperation between an inner covering membrane and an outer covering membrane.
FIG. 32 is a schematic view of a third guide structure.
FIG. 33 is a schematic view showing cooperation of different guide structures.
FIG. 34 is a schematic structural view of an artificial implant according to an embodiment.
FIG. 35a is an enlarged view of a portion of the sealing assembly of the artificial implant of FIG. 34.
FIG. 35b is a schematic view of an embodiment with a first guide structure relative to FIG. 35a.
FIG. 35c is a schematic view of a modified first guide structure relative to FIG. 35b.
FIGS. 36 to 41 are schematic views of the sealing assembly and actuator in different states from different perspectives.
FIGS. 42 to 48 are schematic views of actuator arrangements according to different embodiments.
FIG. 49 is a schematic structural view of an artificial implant according to another embodiment.
FIG. 50 is a cross-sectional view of the artificial implant of FIG. 49.
FIGS. 51 and 52 are schematic views of the sealing assembly and actuator in different states according to yet another embodiment.
FIG. 53 is a schematic view of the loading or retrieval process of an artificial implant without an actuator.
FIG. 54 is a schematic view of the loading or retrieval process of an artificial implant with an actuator.

The reference numerals in the figures are set forth as follows:
400 - artificial implant; 401 - inflow side; 402 - outflow side; 403 - blood flow channel;410 - stent; 411 - inner frame; 4111 - wide region; 412 - outer framework; 4131 - first connection point; 4132 - second connection point; 4133 - third connection point; 4134 - limiting structure; 4135 - connection hole; 420 - sealing assembly; 421 - first guide structure; 4210 - slit; 4212 - groove; 4213 - reinforced portion; 422 - sealing block; 423 - reinforcing wire; 430 - leaflet; 440 - inner covering membrane; 441 - second guide structure; 442 - protrusion; 450 - outer covering membrane; 451 - third guide structure; 460 - actuator; 4601, 4602, 4603 - pulling wire; 461 - protective sleeve; 462 - threading ring; 470 - outer catheter.

### DETAILED DESCRIPTION

The technical solutions of the embodiments of the present application will be described clearly and completely below with reference to the accompanying drawings of the embodiments of the present application. It is apparent that the described embodiments are only a part of, and not all of, the embodiments of the present application. Based on the embodiments of the present application, all other embodiments obtained by those of ordinary skill in the art without creative efforts shall fall within the scope of protection of the present application.

It should be noted that when a component is referred to as being "connected" to another component, it may be directly connected to the other component or an intervening component may also be present. When a component is considered to be "disposed on" another component, it may be directly disposed on the other component or an intervening component may also be present.

Unless otherwise defined, all technical and scientific terms used herein have the same meaning as commonly understood by one of ordinary skill in the art to which this application belongs. The terminology used herein in the description of the application is for the purpose of describing particular embodiments only and is not intended to limit the application. As used herein, the term "and/or" includes any and all combinations of one or more of the associated listed items.

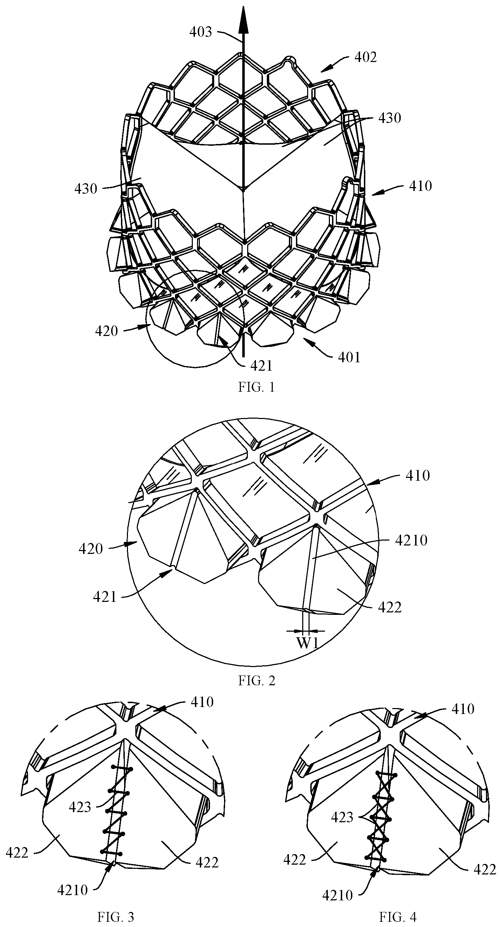

Referring to FIG. 1, the present application discloses an artificial implant, comprising:
a stent 410 having a tubular structure, wherein the tubular structure is radially deformable and consequently has a corresponding compressed state and expanded state; and
a sealing assembly 420 connected to the stent 410 and arranged circumferentially around the stent 410, wherein when the stent 410 is in the expanded state, at least a part of the sealing assembly 420 protrudes outward from an outer periphery of the stent 410, and when the stent 410 is in the compressed state, the sealing assembly 420 is guided to fold to accommodate the deformation of the stent 410.

In order to adapt to the deformation of the stent 410 and to achieve a more regular shape change of the sealing assembly 420 when the stent 410 is in the compressed state, the present application guides the deformation of the sealing assembly 420. The main driving force for the guidance can come from the deformation of the stent 410, which may act directly or indirectly on a predetermined portion of the sealing assembly 420 to promote folding or achieve a predetermined folded shape. When folded, at least a part of the sealing assembly 420 moves radially inward of the stent 410, for example, the portion that protrudes outward in the expanded state moves radially inward, so as to fully utilize the interior space of the stent, minimize the radial dimension for loading and transcatheter delivery, and avoid the stent 410 pinching or crushing the sealing assembly 420 after switching to the compressed state, reducing the risk of damage to the sealing assembly 420.

In some embodiments, the sealing assembly 420 has a first guide structure 421, and when the stent 410 is in the compressed state, the sealing assembly 420 folds at the first guide structure 421. Since the first guide structure 421 is primarily a structural feature of the sealing assembly 420 itself, it can be understood that the deformation of the stent 410 directly acts on the sealing assembly 420 to guide folding; of course, the location of the first guide structure 421 is the predetermined portion of the sealing assembly 420.

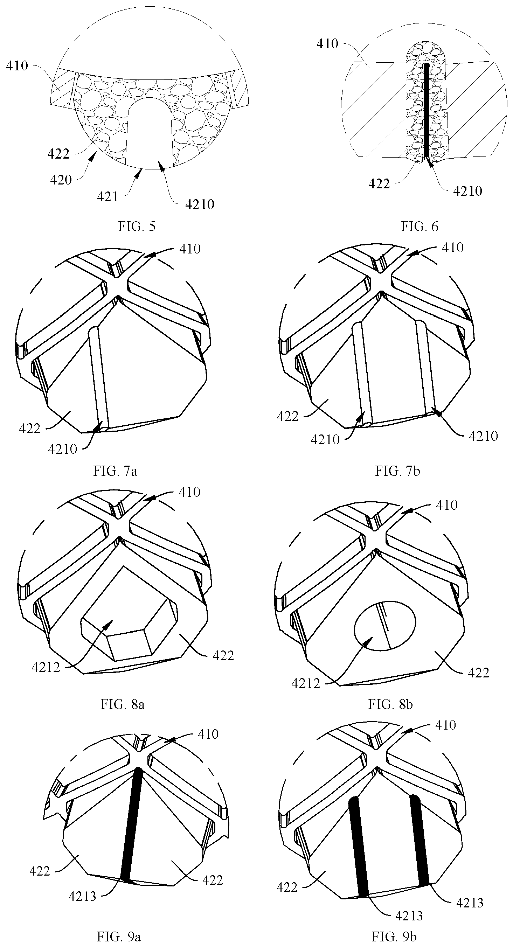

The sealing assembly 420 achieves paravalvular leakage prevention by protruding outward relative to the stent 410. As shown in FIGS. 5 and 6, the first guide structure 421 (specifically, a slit 4210 as an example) can improve the adaptability of the sealing assembly 420 to different states of the stent 410. For example, the first guide structure 421 is a stress-weak structure, so that when the sealing assembly 420 follows the state change of the stent 410, the first guide structure 421 or the portion where it is located deforms more easily than other portions; for example, the first guide structure 421 is a clearance structure, providing deformation space for the sealing assembly 420 during state change; for example, the first guide structure 421 is a reinforced portion, driving other portions to deform. In principle, the first guide structure 421 can guide the deformation process of the sealing assembly 420.

The stent 410 has a grid structure. The grid structure is used to achieve switching between the compressed and expanded states. The stent 410 has an inflow side 401 and an outflow side 402 opposite each other in the axial direction under blood flow environment, the interior of the stent 410 defines a blood flow channel 403, and the artificial implant further comprises leaflets 430 connected to the stent 410 for controlling the blood flow channel 403. The leaflets 430 comprise 2, 3 or 4 leaflets cooperating with each other. The leaflets 430 are made of a biological material or a polymer material. The sealing assembly 420 is located on the inflow side 401 of the leaflets 430.

As shown in FIGS. 5 to 8b, the first guide structure 421 comprises at least one weakened portion, and when the stent 410 is in the compressed state, the sealing assembly 420 folds at the first guide structure 421, thereby being radially inwardly collapsible toward the stent 410. The sealing assembly 420 comprises a plurality of deformable sealing blocks 422 arranged at intervals. In one embodiment, the sealing blocks 422 are made of PU foam. Due to the compressibility and self-expandability of PU foam, when subjected to uneven external forces, it can automatically adjust its height and thickness, while absorbing blood to self-expand, closely fitting the annulus to achieve paravalvular leakage prevention.

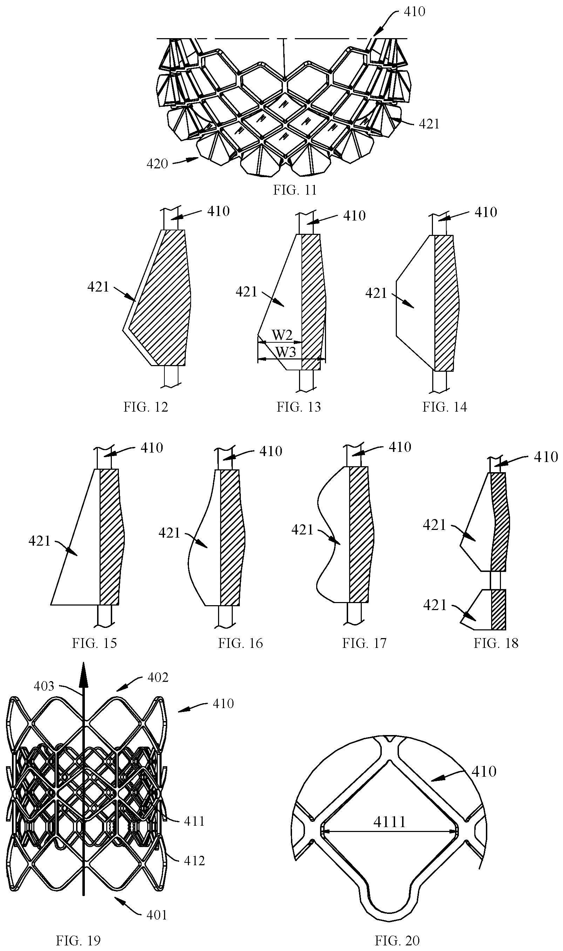

As shown in FIGS. 2 to 6, the sealing blocks 422 can have various shapes. In an axial view of the stent, the projected shape of the sealing block 422 may be triangular, diamond-shaped, semi-circular, semi-elliptical, rectangular, polygonal, etc. Similarly, in a circumferential view of the stent, the projected shape of the sealing block 422 may be triangular, diamond-shaped, semi-circular, semi-elliptical, rectangular, polygonal, etc. In terms of cross-sectional shape, referring to FIGS. 12 to 18, various forms of sealing blocks are disclosed, mainly reflecting different externally protruding shapes relative to the stent. In FIGS. 12, 13 and 15, the protruding external shape of the sealing block is triangular, with different apex positions; in FIG. 14, the protruding external shape is trapezoidal; in FIG. 16, the protruding external shape is an irregular curved surface, with the side near the inflow side protruding more than the side near the outflow side; in FIG. 17, the protruding external shape is B-shaped; in FIG. 18, sealing blocks are arranged in cooperation with each other in the axial direction, each having a triangular protruding shape, forming a separated B-shape together.

As shown in FIG. 2, the weakened portion of the first guide structure 421 is a slit 4210 formed in the sealing block 422. The slit 4210 extends in a direction parallel to a generatrix of the stent 410. The provision of the slit 4210 releases the deformation stroke of the sealing blocks 422 on both sides. The width of the slit 4210 can adjust the above effect. For example, in FIG. 5, the width of the slit 4210 matches the width of the strut of the stent 410. As shown in other figures, the width W1 of the slit 4210 is 0~1 mm. When the width is 0 mm, the sealing block 422 has a fractured region structurally, but the two sides of the fractured region abut each other. In addition to the width, the depth of the slit 4210 can also adjust its working effect. For example, in FIG. 12, the first guide structure 421 extends along the surface of the sealing block 422. In FIG. 13, the extension direction of the first guide structure 421 is parallel to the generatrix of the stent. Moreover, the depth of the first guide structure 421 can also adjust its working effect. For example, in FIG. 12, in the radial direction of the stent 410, the depth of the first guide structure 421 is less than the width of the stent, i.e., a shallow groove. In FIG. 13, in the radial direction of the stent 410, the depth W2 of the first guide structure 421 is at least half of the thickness W3 of the sealing block 422.

As also shown in FIG. 3, in the radial direction of the stent 410, the slit 4210 as the first guide structure extends through the sealing block 422, and the sealing block 422 is maintained connected on both sides of the slit 4210 by a first component. For example, the first component is a reinforcing wire 423 threaded through the sealing block 422. The reinforcing wire 423 may also be formed into a reinforcing fabric by various weaving forms.

The sealing block 422 may be provided with a reinforcing wire 423 that is pulled on both sides of the slit 4210. In FIG. 3, for the same slit 4210, the reinforcing wire 423 is a single thread reciprocally threaded through both sides of the slit. In FIG. 4, for the same slit 4210, there are a plurality of reinforcing wires 423, and each reinforcing wire forms knots independently or in combination with other reinforcing wire. There are a plurality of knots arranged at intervals along the extension direction of the slit 4210. In detail, the threading path of the reinforcing wire 423 avoids the stent 410 or wraps around the stent 410. The reinforcing wire 423 is fixed by tying or by an additional connector member.

As shown in FIG. 8a or 8b, the weakened portion of the first guide structure 421 is a groove 4212 formed in the sealing block 422. The open side of the groove 4212 faces radially outward of the stent 410. The depth of the groove 4212 is at least half of the thickness of the sealing block 422. Similarly, the groove 4212 may extend through the sealing block 422, and the sealing block 422 itself forms an annular structure. The groove 4212 may be located at a geometric center of the sealing block 422. The cross-sectional shape of the groove 4212 may match the shape of the sealing block, as shown in FIG. 8a; or may be independently configured as a regular geometric shape, such as a circle, ellipse, triangle, diamond, rectangle, polygon, etc., as shown in FIG. 8b. Different parts of the sealing block 422 have different thicknesses in the radial direction of the stent 410, and the slit 4210 intersects at least the portion having the greatest thickness. In FIG. 8a or 8b, the slit 4210 extends axially through the sealing block 422. In other embodiments, at least one side of the slit 4210 is closed.

There are various combinations of sealing blocks 422 and first guide structures 421. For example, in FIG. 2, the first guide structure 421 passes through the geometric center of the sealing block 422. In FIG. 7a, the first guide structure 421 is eccentrically disposed relative to the geometric center of the sealing block 422. In FIG. 7b, the same sealing block 422 has a plurality of first guide structures 421. The plurality of first guide structures 421 are symmetrically or asymmetrically arranged relative to the geometric center of the sealing block 422.

As shown in FIGS. 9a to 9b, the first guide structure 421 comprises at least one reinforced portion 4213, and the reinforced portion 4213 drives other portions of the sealing assembly to fold and collapse radially inward of the stent 410. In this embodiment, the reinforced portion 4213 drives other portions of the sealing block 422 where it is located to fold and collapse radially inward of the stent 410.

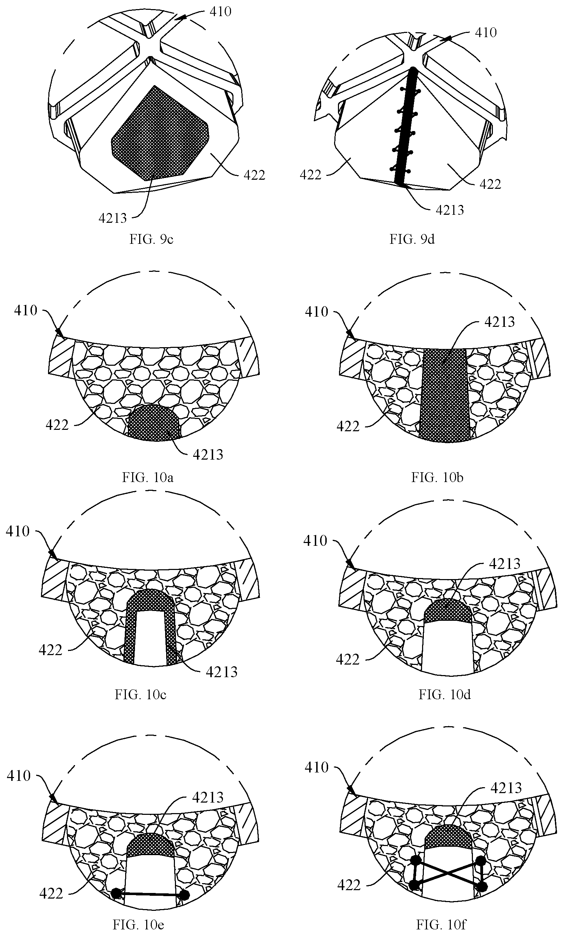

In terms of implementing the reinforced portion 4213, it can be formed by increasing the strength of a partial region of the sealing block 422, i.e., the reinforced portion 4213 and the sealing block 422 are integral. This can be further divided into same-material and different-material configurations. For example, when the reinforced portion 4213 and the sealing block 422 are integral and made of the same material, the reinforced portion can be achieved by increasing the material density of a partial region, increasing the thickness of a partial region, or increasing the elasticity and/or yield strength of the material of a partial region. For example, when the reinforced portion 4213 and the sealing block 422 are integral but made of different materials, the reinforced portion can be achieved by coupling a material with different elasticity and/or yield strength in a partial region. As shown in FIG. 9d, the reinforced portion 4213 may also be formed by another component, i.e., the reinforced portion 4213 and the sealing block 422 are separate components. The first component may be a reinforcing wire threaded through the sealing block 422. In the embodiment where a reinforcing wire forms the reinforced portion 4213, the reinforced portion 4213 may be the reinforcing wire itself, or may be a local material stack caused by the threading of the reinforcing wire, thereby achieving local reinforcement. That is, the reinforcing wire can independently form the reinforced portion, or can cooperate with other methods to form the reinforced portion.

Similarly to the weakened portion described above, the position, shape, and relationship with surrounding components of the reinforced portion 4213 can adjust its guiding effect on the sealing block. As shown in FIG. 9a, the reinforced portion 4213 is located at the middle of the sealing block and extends along the generatrix of the stent. As shown in FIG. 9b, a plurality of reinforced portions 4213 are provided and are symmetrically arranged relative to the geometric center of the sealing block. As shown in FIG. 9c, the reinforced portion 4213 matches the outer peripheral shape of the sealing block. As shown in FIG. 10a, the depth of the reinforced portion 4213 is part of the thickness of the sealing block 422. As shown in FIG. 10b, the reinforced portion 4213 extends through the sealing block 422, i.e., its depth is the entire thickness of the sealing block 422.

The weakened portion and the reinforced portion 4213 can also be arranged in combination. As shown in FIG. 10c, the first guide structure 421 comprises at least one weakened portion and a reinforced portion 4213 acting on the weakened portion, and the sealing assembly folds at the at least one weakened portion, thereby being radially inwardly collapsible toward the stent 410. The two cooperate to guide the deformation process of the sealing block. The reinforced portion 4213 may be provided outside or at the edge of the weakened portion. Alternatively, as shown in FIG. 10c, the weakened portion forms a cavity region, and the reinforced portion 4213 is disposed around an inner circumferential surface of the cavity region. In FIG. 10d, the weakened portion forms a cavity region, and the reinforced portion 4213 is located on an inner circumferential surface of the cavity region near the inner side of the stent, i.e., at the bottom of the weakened portion.

Furthermore, the weakened portion and the reinforced portion 4213 can also cooperate with a reinforcing wire. As shown in FIG. 10e, both sides of the weakened portion are connected by a reinforcing wire. The reinforcing wire can further adjust its working effect through different threading methods. In FIG. 10f, the reinforcing wire extends into the interior of the weakened portion.

The first guide structures 421 may be the same or different. Multiple sealing blocks 422 may be uniformly configured, i.e., all sealing blocks 422 have the first guide structures 421, and the first guide structures 421 are the same. Multiple sealing blocks 422 may also be configured differently, e.g., only some of the sealing blocks 422 have the first guide structures 421, or all sealing blocks 422 have the first guide structures 421 but at least one first guide structure 421 is different.

Overall, the sealing blocks 422 made of PU porous foam material optimize the skirt design of the artificial implant, utilizing the compressibility of the foam to avoid increasing the delivery system profile. The special shape design of the first guide structure 421 enables orderly folding of the foam region of the sealing block 422, further reducing the delivery profile of the valve, reducing the risk of breakage or detachment of the sealing block 422 during loading and retrieval, while not affecting the paravalvular leakage prevention effect.

In a specific configuration, the stent 410 is self-expandable. The stent 410 is made of a memory material, such as a nickel-titanium alloy. In other embodiments, the stent 410 is balloon-expandable. The material of the stent 410 can meet the performance requirements for balloon expansion. As shown in FIG. 19, the stent 410 comprises an inner frame 411 defining the blood flow channel 403 and an outer framework 412 connected to the inner frame 411 and positioned around the outer periphery of the inner frame 411 in the expanded state, and the outer framework 412 is made of a memory material suitable for self-expansion. The outer framework 412 comprises arms arranged at intervals in the circumferential direction and/or an outer frame sleeved radially outside the inner frame 411.

The stent 410 is formed by cutting or braiding and has a plurality of grids, and during transition of the stent 410 state switching, some or all of the grids deform adaptively. The sealing blocks 422 are embedded in corresponding grids.

The sealing blocks 422 are arranged in one or more groups along the axial direction of the stent 410, and sealing blocks in the same group are distributed circumferentially in the same ring of grids. The sealing blocks 422 may fill the entire grid, or may only occupy a portion of the grid. As shown in FIG. 20, the grid has a wide region 4111 with the largest span in the circumferential direction of the stent 410, and the first guide structure 421 intersects the wide region 4111. The sealing block 422 at least fills the wide region 4111. The extension direction of the first guide structure 421 is perpendicular to the extension direction of the wide region 4111. In FIG. 2, in the circumferential direction of the stent 410, the first guide structure 421 is located at a middle portion of the sealing block 422. In FIG. 7a, in the circumferential direction of the stent 410, the first guide structure 421 is offset to one side. When the first guide structure 421 is offset, the first guide structures 421 on multiple sealing blocks 422 may have independently set offset directions, or may all be offset in the same circumferential direction. The offset amount of each first guide structure 421 may be set independently or uniformly.

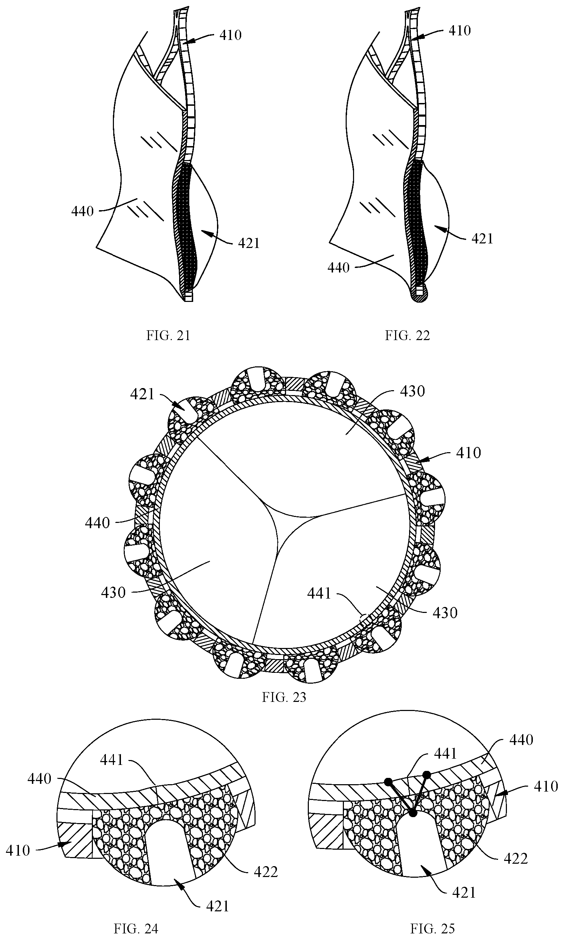

Referring to FIG. 21, the artificial implant further comprises:
An inner covering membrane 440 distributed around an inner circumferential wall of the inner frame 411, the sealing assembly 420 being fixed to an outer side of the inner covering membrane 440, and when the stent 410 is in the expanded state, the sealing assembly 420 protrudes outward of the stent 410 through corresponding grids. In one embodiment, the inner covering membrane 440 is a PU film. Similar to the advantages of PU foam mentioned above, PU film also has good elasticity and adaptability to inward folding. A method for preparing the PU film can refer to the following:
Dissolving implantable PU particles (i.e., polyurethane) in a DMF solvent to obtain a PU solution (polyurethane solution) with a polyurethane content of 5% to 20% (w/v); when dissolving the PU particles, using a mechanical stirrer to accelerate dissolution, at room temperature or with heating (18-90°C);
Pouring a certain amount of the PU solution into a flat mold, placing the flat mold in an oven at 30-70°C for 7-24 hours to obtain the PU film, the thickness of which can be adjusted according to the solution concentration and the height of the solution after pouring, with a minimum thickness of 0.02 mm.

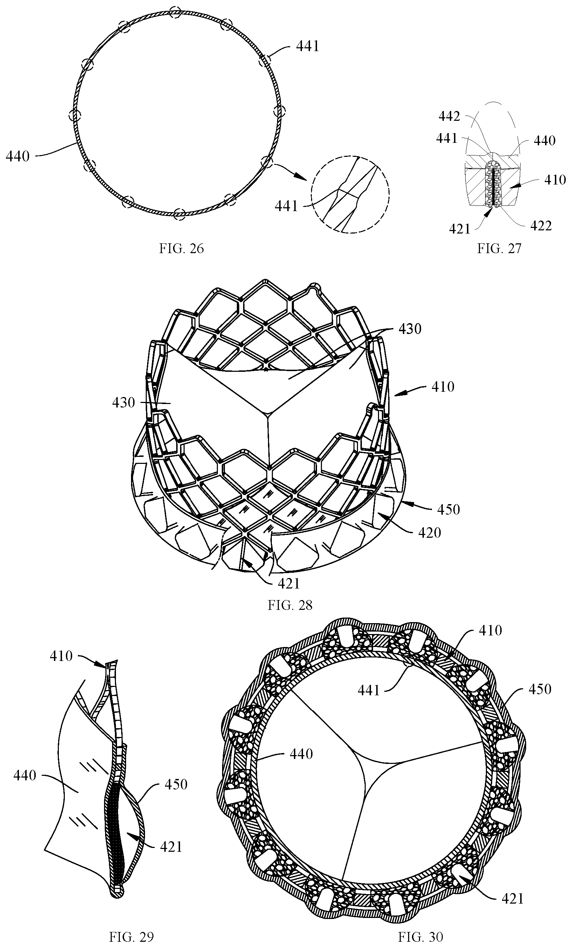

As shown in FIG. 27, when the stent 410 is in the compressed state, the folded portion of the sealing assembly 420 together with the corresponding portion of the inner covering membrane 440 forms an inward protrusion 442 relative to the inner circumferential wall of the stent 410. In an axial view of the stent 410, at least 30% of the sealing assembly 420 is located in the inward protrusion 442. The inner covering membrane 440 is bonded to the sealing assembly 420, or the sealing assembly 420 is formed directly on the surface of the inner covering membrane 440. In terms of forming methods, the two can be produced separately and then assembled, or can be integrally processed.

For example, a method for integrally forming a PU film and PU foam is as follows:
Dissolving implantable PU particles in a DMF solvent to obtain a PU solution (PU particle content 5%-20% (w/v)), mixing the PU solution with sodium chloride to obtain a premix for foam preparation, where the diameter of the sodium chloride is about 5-500 micrometers, and the mass fraction of sodium chloride in the premix is 50%-80%;
Filling the premix into a mold and compacting, the shape of the mold can be varied according to the design of the sealing assembly;
Heating the PU film (40-80°C), then spreading it flat on the mold surface, allowing it to stand or placing it in an oven for a certain time;
After demolding, continuing to dissolve the salt particles in water, and drying to obtain the integrally formed PU foam and PU film material.

In the embodiments shown in FIGS. 23 to 27, in the embodiment where the inner covering membrane 440 has a second guide structure 441, the second guide structure 441 can be formed simultaneously during the processing of the inner covering membrane 440. For example, replacing the flat mold for containing the PU solution with a pre-shaped forming mold, the PU film obtained from that forming mold already has the second guide structure 441. The second guide structure 441 can also be formed independently of the processing of the inner covering membrane 440. For example, a flat inner covering membrane 440 is formed into a pre-shaped shape and placed in an oven at a preset temperature for a preset time to obtain the inner covering membrane 440 with the second guide structure 441. In terms of specific structural embodiment, the second guide structure 441 has various forms, such as a three-dimensional structure protruding or recessed relative to the plane of the inner covering membrane 440; or a region that is weaker or stronger than the surrounding area; etc. Exemplarily, in FIG. 23, the second guide structure 441 is a crease on the inner covering membrane 440.

The sealing assembly and inner covering membrane are bonded or integrally processed, which can achieve a high peel strength, so that when the sealing assembly is exposed, even if it contacts and rubs against the catheter assembly or body tissue, it is not easily detached, avoiding the safety risk of detachment of the sealing assembly.

In terms of the cooperative relationship of the inner covering membrane 440 with other components, the outflow side 402 of the inner covering membrane 440 is spliced with the leaflets 430 and fixed to the stent 410. In FIG. 21, the inflow side 401 of the inner covering membrane 440 extends to the inflow side end of the stent 410. In FIG. 22, the inflow side 401 of the inner covering membrane 440 is everted to wrap around the inflow side end of the stent 410. The eversion wrapping may be complete wrapping or intermittent partial wrapping. In embodiments where the inner covering membrane 440 is everted to wrap around the stent 410, the everted portion of the inner covering membrane 440 may extend radially inside the sealing assembly, or may cover the radially outer side of the sealing assembly. When the everted portion of the inner covering membrane 440 covers the radially outer side of the sealing assembly, the everted portion can be understood as an outer covering membrane 450 described below.

As shown in FIGS. 23 to 27, the inner covering membrane 440 has a second guide structure 441. The second guide structure 441 corresponds in position to the first guide structure 421 in the circumferential direction of the stent 410. Regarding the cooperative relationship between the second guide structure 441 and the sealing block 422, as shown in FIG. 24, the shapes of the two cooperate with each other. As shown in FIG. 25, the two are independently arranged. The second guide structure 441 may be oriented radially inward of the stent 410, or as shown in FIG. 26, oriented radially outward of the stent 410, or bidirectional. In FIG. 25, a connecting member is provided between the second guide structure 441 and the sealing block 422, which may be a reinforcing wire 423 or a reinforcing fabric. The connection position of the connecting member with the sealing block 422 may be inside the first guide structure 421 or at another location.

As shown in FIG. 28, the artificial implant further comprises: an outer covering membrane 450 distributed around an outer circumferential wall of the stent 410, wherein part or all of the sealing assembly 420 is wrapped by the outer covering membrane 450.

The outer covering membrane 450 may be independently provided, or as shown in FIG. 29, the outer covering membrane 450 and the inner covering membrane 440 are integrally formed. When the outer covering membrane 450 and the inner covering membrane 440 are integrally formed, the outer covering membrane 450 can be understood as formed by everting the inner covering membrane 440 to wrap around the inflow side end of the stent 410 and extending over the outer peripheral surface of the sealing assembly 420. As shown in FIGS. 29 and 30, the outer covering membrane 450 and the inner covering membrane 440 together define a pocket structure having a radial gap, the sealing assembly 420 is fixed to the outer surface of the inner covering membrane 440, and the entire sealing assembly 420 is located within the pocket structure, or the outflow side 402 of the sealing assembly 420 is exposed outside the pocket structure.

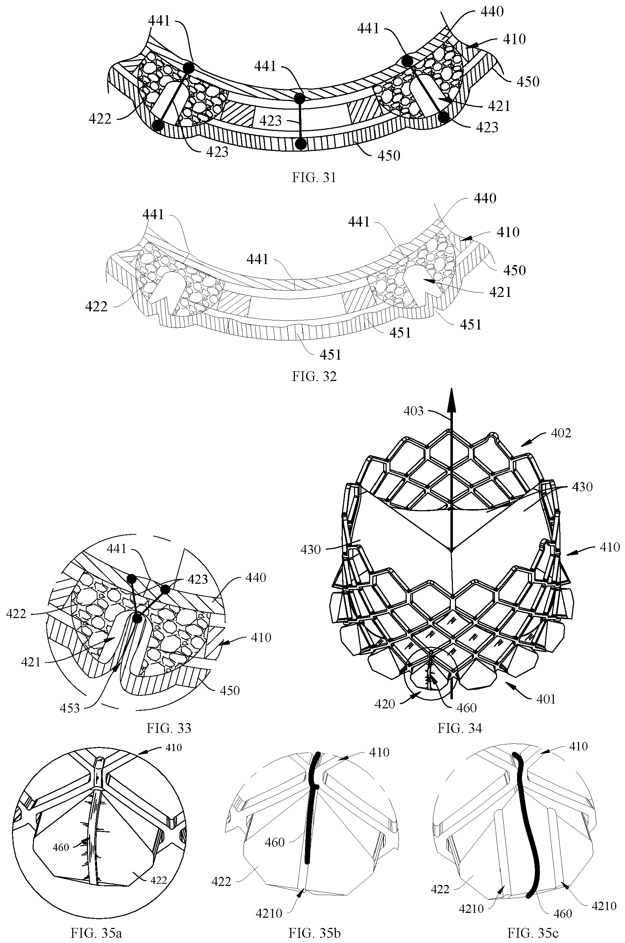

The outer covering membrane 450 may be provided independently of the inner covering membrane 440 or the sealing block 422, or as shown in FIG. 31, the second guide structure 441 is connected to the outer covering membrane 450. The connection may be achieved by a reinforcing wire 423 or a reinforcing fabric.

As shown in FIG. 32, the outer covering membrane 450 has a third guide structure 451. The third guide structure 451 may be oriented radially inward of the stent 410, or radially outward of the stent 410, or bidirectional. The positions of the third guide structure 451 and the first guide structure 421 may correspond to each other, for example, the third guide structure 451 is embedded in the first guide structure 421. The outer covering membrane 450 has an axial width corresponding to at least one complete ring of grids, and the third guide structure 451 is located at a midline portion of the corresponding grids.

As shown in FIG. 33, the third guide structure 451 is connected to the inner covering membrane 440. The connection may be by bonding or suturing. When suturing is used, it may be achieved by a reinforcing wire 423 or a reinforcing fabric. In FIG. 33, the third guide structure 451 is embedded in the first guide structure 421. During embedding, the outer covering membrane 450 is partially folded to form the third guide structure 451. The size of the third guide structure 451 is smaller than that of the first guide structure 421 to ensure normal operation of the first guide structure 421. The embedded portion is fixedly connected to the sealing assembly 420 and/or the inner covering membrane 440. The third guide structure 451 is fixedly connected to the sealing assembly 420 and/or the inner covering membrane 440 at least at the outflow side edge portion of the outer covering membrane 450. In terms of the connection position, the reinforcing wire 423 may be connected to both ends of the second guide structure 441 to ensure normal operation of the second guide structure 441.

In terms of the embodiment of each guide structure, the inner covering membrane 440 has a second guide structure 441, and the outer covering membrane 450 has a third guide structure 451, each of the second and third guide structures independently being a low-strength band or a pre-formed crease. The low-strength band has a smaller thickness than adjacent portions or is made of a different material. The pre-formed crease is obtained by reciprocally folding and then heat-treating the material. The low-strength band or pre-formed crease may also be obtained by the preset shape of the mold during production. The second guide structure 441 and the third guide structure 451 are respectively arranged at intervals in the circumferential direction along the stent 410. The number of the second guide structures 441 and the third guide structures 451 is equal to the number of grids in the axial direction of the stent 410, or is an integer multiple thereof.

Based on the above features of the first guide structure, an embodiment of the present application also discloses an artificial implant, comprising:
a stent having a tubular structure, wherein the tubular structure is radially deformable and consequently has a corresponding compressed state and expanded state; and
a sealing assembly connected to the stent and arranged circumferentially around the stent, wherein when the stent is in the expanded state, at least a part of the sealing assembly protrudes outward from an outer periphery of the stent, the sealing assembly has a slit, and when the stent is in the compressed state, the sealing assembly is guided to fold at the slit to accommodate the deformation of the stent.

In order to load or retrieve the artificial implant, an embodiment of the present application also discloses a method for driving deformation of a sealing assembly on an artificial implant, which can be implemented through the guide structures of the above embodiments. For example, the method comprises:
providing an artificial implant, the artificial implant at least comprising a stent and a sealing assembly, the sealing assembly being connected to the stent and arranged circumferentially around the stent, wherein when the stent is in the expanded state, at least a part of the sealing assembly protrudes outward from an outer periphery of the stent;
wherein the sealing assembly has a slit, and when driving the sealing assembly to deform, the sealing assembly is compressed by deformation of the stent so that it is guided to fold at the slit.

From the above description, it is not difficult to understand that an embodiment of the present application also discloses an artificial implant, comprising:
a stent 410 having a tubular structure, wherein the tubular structure is radially deformable and consequently has a corresponding compressed state and expanded state;
an inner covering membrane 440 distributed around an inner circumferential wall of the stent 410, the inner covering membrane 440 being provided with a second guide structure 441, wherein when the stent 410 is in the compressed state, the inner covering membrane 440 folds at the second guide structure 441, thereby being radially inwardly collapsible toward the stent 410; and
an outer covering membrane 450 distributed around an outer circumferential wall of the stent 410, the outer covering membrane 450 being provided with a third guide structure 451, wherein when the stent 410 is in the compressed state, the outer covering membrane 450 folds at the third guide structure 451, thereby being radially inwardly collapsible toward the stent 410.

An embodiment of the present application also discloses an interventional system, comprising:
an artificial implant according to any of the above embodiments, being in a compressed state;
a catheter assembly, wherein at least one tube is inserted through the interior of the artificial implant and connected thereto; and at least one tube wraps around the outer periphery of the artificial implant; and
a control handle connected to and driving the catheter assembly.

As shown in FIGS. 34 to 41, an embodiment of the present application also discloses an artificial implant, comprising:
a stent 410 having a tubular structure, wherein the tubular structure is radially deformable and consequently has a corresponding compressed state and expanded state;
a sealing assembly 420 connected to the stent 410 and arranged circumferentially around the stent 410, wherein when the stent 410 is in the expanded state, at least a part of the sealing assembly 420 protrudes outward from an outer periphery of the stent 410; and
an actuator 460 connected to the stent 410, wherein during transition of the stent 410 from the expanded state to the compressed state, the actuator 460 is driven by the stent 410 to act radially inwardly on at least a part of the sealing assembly 420 to guide folding of the sealing assembly 420. In this embodiment, the deformation of the stent 410 acts indirectly on the sealing assembly 420 through the actuator 460, although the deformation of the stent 410 may also directly compress the sealing assembly 420, but the actuator 460 in this embodiment plays a primary role in guiding the folding.

The actuator 460 may act in a line contact manner to press the sealing assembly 420 into the interior space of the stent 410. Line contact concentrates the acting area, facilitating guided folding. In other embodiments, the contact area between the actuator 460 and the sealing assembly 420 may be increased, for example by surface contact or more dense line contact (e.g., grid contact).

In some embodiments, part or all of the actuator 460 is made of a radiopaque material or has a radiopaque marker, used to indicate the spatial position and orientation of the artificial implant, as well as the morphological changes of the actuator 460 itself and the sealing assembly 420, providing real-time reference during surgery in conjunction with medical imaging equipment.

The stent 410 is self-expandable or balloon-expandable using an inflatable balloon. According to release control requirements, a wire control method may also be combined, i.e., the stent 410 is connected to the interventional delivery system via pull wires, and the release process of the stent 410 can be precisely controlled by tightening or loosening the pull wires at the proximal end, as well as retrieval of the stent 410. In this method, the pull wires can also cooperate with the actuator 460, for example, the pull wires and the actuator 460 are interlocked or even the same component. In this case, the actuator 460 can also be used to drive the deformation of the stent 410.

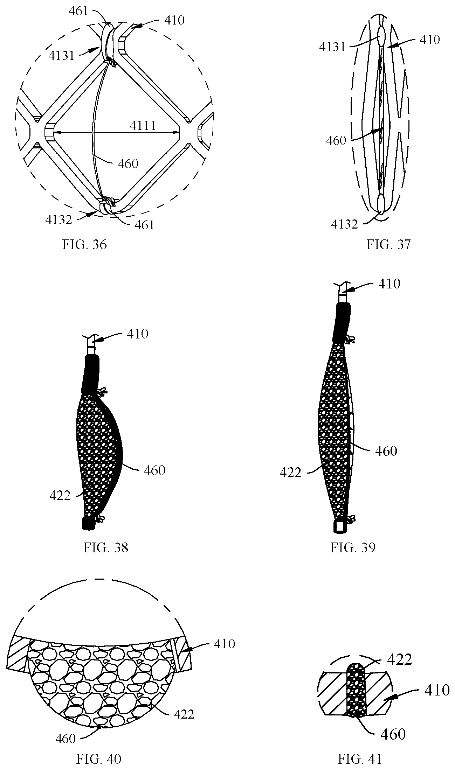

The stent 410 has an inflow side 401 and an outflow side 402 opposite each other in the axial direction under blood flow environment, the interior of the stent 410 defines a blood flow channel 403, and the artificial implant further comprises leaflets 430 connected to the stent 410 for controlling the blood flow channel 403. The leaflets 430 comprise two, three or four leaflets cooperating with each other. The leaflets 430 are made of a biological material or a polymer material. The sealing assembly 420 is located on the inflow side 401 of the leaflets 430. When the stent 410 is in the expanded state, the sealing assembly 420 achieves paravalvular leakage prevention by protruding outward relative to the stent 410 (as shown in FIGS. 38 and 40). When the stent 410 is in the compressed state, the sealing assembly 420 is compressed to adapt to the shape of the stent 410 (as shown in FIGS. 39 and 41). During compression, it may be assisted by the actuator 460, which can also respond to state changes of the stent (as shown in FIGS. 36 and 37).

The actuator 460 compresses the sealing assembly 420 by acting radially inwardly on at least a part of the sealing assembly 420. In some embodiments, the acting portion preferably passes through the geometric center of the radial projection of the sealing assembly 420. As shown in FIG. 36, when the stent 410 has a grid structure, the acting portion passes through the wide region 4111 of the grid having the largest circumferential dimension.

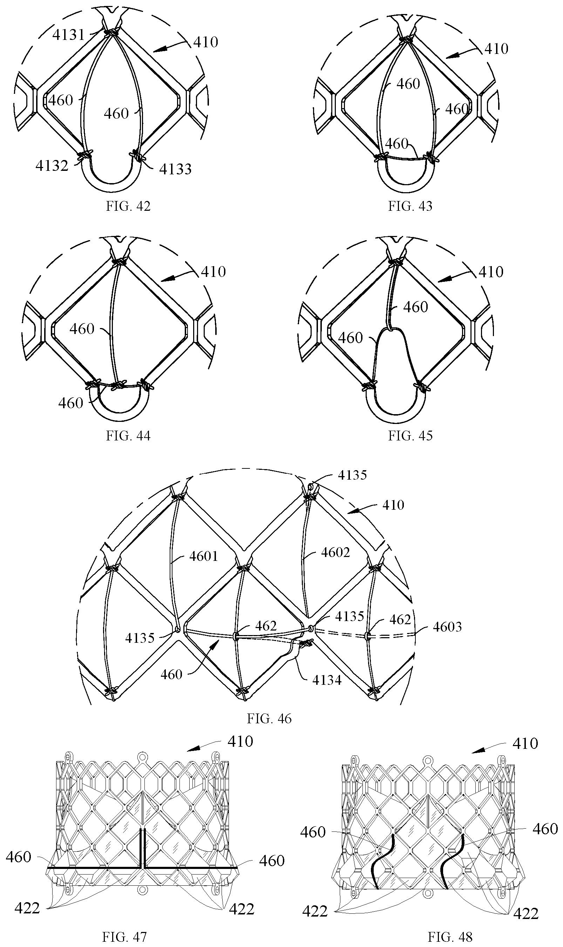

When the actuator 460 acts in a line contact manner, as shown in FIG. 36 or 42, the line contact form is a straight line (or curve); as shown in FIGS. 43 and 44, the line contact form is crossed; as shown in FIG. 45, the line contact form is radial; as shown in FIG. 46, the line contact form is grid-like.

The stent 410 has a grid structure, and during transition of the stent 410 between states, the axial length of at least one grid of the grid structure changes correspondingly, and the actuator 460 is configured to be driven by the change in axial length of that grid. As shown in FIG. 48, the actuator 460 may also respond to changes in axial length of multiple grids.

There are various ways to implement the effect of the actuator 460. For example, the actuator 460 is a deformable part or assembly that adjusts its own shape in response to the state of the stent 410. For example, the actuator 460 is a transmission part or assembly that transmits the state change of the stent 410 to the sealing assembly 420.

The actuator 460 comprises at least one pulling wire, both ends of which are constrained by two or more axially spaced portions of the stent 410, wherein in the expanded state the at least one pulling wire is slack to allow the sealing assembly to protrude outward (see FIGS. 36, 38, 40), and in the compressed state the at least one pulling wire is taut to radially inwardly compress the sealing assembly (see FIGS. 37, 39, 41). In FIGS. 36 and 37, for simplicity, the sealing assembly or sealing block is not shown to better illustrate the details of the actuator 460; in FIGS. 38 to 41, the sealing assembly is shown as sealing blocks 422. Preferably, the actuator 460 is made of a polymer wire, a metal wire, or a hybrid wire of polymer and metal. As shown in FIG. 35a, the radial dimension of the actuator 460 is close to the radial dimension of the strut of the stent to withstand greater torque. As shown in FIG. 36, the radial dimension of the actuator 460 is smaller than the radial dimension of the strut of the stent to concentrate stress and optimize the compression effect.

The sealing assembly 420 comprises a plurality of deformable sealing blocks 422 arranged at intervals. In terms of cooperation with the sealing assembly 420, as shown in FIG. 35a, the actuator 460 drives a single sealing block 422, which allows precise control of the state change of the sealing block 422.

As shown in FIG. 35b, the first guide structure mentioned in the above embodiments can also be used in combination with the actuator 460, for example, the first guide structure 421 corresponds in position to the actuator 460. When the first guide structure is a slit 4210, at least a part of the actuator 460 (shown as a thick black line in the figure) can extend through the slit 4210, and both ends of the actuator 460 can be tied to the stent 410.

As shown in FIG. 35c, the actuator 460 may also be arranged offset from the first guide structure, cooperating from different positions to guide the folding of the sealing block 422. For example, there are two slits 4210 arranged circumferentially on both sides of the actuator 460 (shown as a thick black line in the figure).

As also shown in FIG. 47, the actuator 460 synchronously drives multiple sealing blocks 422. This configuration reduces the number of moving parts and optimizes the overall state control of the artificial implant. To better control the sealing blocks 422, the sealing blocks 422 fill the grid and, within a single grid, the pulling wire passes through the geometric center of the grid. The above methods can also be combined. As shown in FIG. 46, part of the actuator 460 drives a single sealing block (for simplicity, the sealing assembly or sealing block is not shown in FIG. 46 to better illustrate the details of the actuator 460), and another part synchronously drives multiple sealing blocks.

In terms of cooperation with the stent 410, as shown in FIG. 37, the stent 410 has a first connection point 4131 and a second connection point 4132 spaced apart in the axial direction. Each connection point is selected from a location suitable for restraining the actuating member.

The actuator 460 is a flexible member or a deformable structure, with at least one end connected to the first connection point 4131 and at least the other end of the actuator connected to the second connection point 4132. For example, in the embodiment shown in FIGS. 36 and 37, the first connection point 4131 and the second connection point 4132 are respectively located at grid vertices of the stent 410. In another example, as shown in FIG. 42, the stent 410 has a first connection point 4131 and a second connection point 4132 and a third connection point 4133 spaced apart in the axial direction, where the first connection point 4131 is at a grid vertex of the stent 410, and the second connection point 4132 and the third connection point 4133 are on struts of the stent 410, which may be aligned or offset circumferentially.

It can be understood that the connection points need to provide a certain limiting effect. For example, in FIG. 36, the connection points are grid vertices of the stent 410; in FIG. 46, the connection points are connection holes 4135 formed in the stent 410; in FIG. 46, the connection points are notches or wave-shaped limiting structures 4134, etc. From the above description, it can be seen that the function of the connection points is to limit the positional relationship between the actuator 460 and the stent 410, but not necessarily to fix the spatial position of the actuator 460. For example, in FIG. 46, the connection points are in the form of connection holes 4135 and the actuator 460 is a pulling wire, which can be movably threaded through the connection holes 4135 to adjust its axial position. In another example, in FIGS. 42 to 45, the actuator 460 is tied to the connection points of the stent 410, thereby limiting its spatial position and achieving a fixed connection. Therefore, in terms of connection methods, the end of the actuator 460 is connected to the stent 410 and/or to another actuator 460 by one or more of tying (as shown in FIG. 42), threading (as shown in FIG. 46, where the stent 410 has connection holes 4135), wrapping (threading through structural gaps of the stent 410 itself), bonding, and welding.

As shown in FIG. 36, the actuator 460 is connected to the stent 410 via a protective sleeve 461 to reduce wear and disperse stress. The protective sleeve 461 and/or the actuator 460 may be made of a wear-resistant and/or self-lubricating polymer material. In the embodiments shown in the figures, the actuator 460 comprises a plurality of pulling wires arranged in various ways.

For example, as shown in FIG. 42, a plurality of pulling wires extend in the axial direction of the stent 410. Axially extending pulling wires can respond to changes in the axial length of the stent 410, thereby achieving a driving effect. A plurality of pulling wires arranged in parallel can improve driving stability.

For example, as shown in FIGS. 43 to 45, at least one pulling wire extends in the circumferential direction of the stent 410. Pulling wires can cooperate with each other to achieve the effect on the sealing assembly. For example, in FIG. 43, a circumferentially extending pulling wire can limit the lower ends of two axially extending pulling wires, so that even if connected to smoothly curved struts, the positions of the pulling wires can be stably set. For example, in FIG. 44, the circumferentially extending pulling wire, in addition to determining the lower ends of the axially extending pulling wires, can also participate in responding to state changes of the stent 410 together with the axially extending pulling wires to compress the sealing assembly. It can be understood that the circumferentially extending pulling wire can adjust its length to change the cooperation position with the axially extending pulling wires (as shown in FIG. 45).

For example, as shown in FIG. 46, a part of at least one pulling wire extends in the axial direction of the stent 410, and another part extends in the circumferential direction of the stent 410. In this embodiment, the circumferentially extending part, in addition to cooperating with the axially extending part to respond to state changes of the stent 410, can also provide a structural basis for synchronizing multiple sealing blocks.

It can also be understood that at least one pulling wire may extend simultaneously in the axial and circumferential directions of the stent 410, for example, the pulling wire extends obliquely relative to the axis of the stent.

The above arrangements can be implemented independently or in combination.

When there are a plurality of pulling wires, they may be arranged independently or in cooperation with each other. In the embodiment shown in FIG. 46, within at least one grid, a pulling wire extending in the axial direction of the stent 410 and a pulling wire extending in the circumferential direction cross each other. A crossing point is near the geometric center of the grid. The two pulling wires may be fixed relative to the crossing point to increase load strength. Alternatively, as shown in FIG. 46, the two pulling wires may be movable relative to the crossing point. One of the pulling wires has a threading ring 462 for another pulling wire to pass through. In terms of forming the threading ring 462, it may be formed by looping the pulling wire itself, or the threading ring 462 may be independently provided and connected to the corresponding pulling wire. For example, the threading ring 462 is a separate polymer ring or metal ring. The connection between the threading ring 462 and the pulling wire may be achieved by threading, welding, bonding, wrapping, etc.

In the embodiment shown in FIG. 46, each grid provided with the sealing assembly 420 corresponds to at least one pulling wire, and the number of pulling wires in each grid provided with the sealing assembly 420 may be the same or different. When calculating the number of pulling wires in each grid provided with the sealing assembly 420, only those pulling wires that cooperate with the sealing assembly 420 in that grid should be considered; pulling wires that merely pass through the grid without cooperating with the sealing assembly 420 in that grid should not be counted.

There are various forms of the extension path of the pulling wire. As shown in FIG. 46, after the pulling wire 4601 extends axially from a grid vertex of the stent 410, passes through the left connection hole 4135, and then extends circumferentially through the threading ring 462, it may be arranged as follows:
Method 1: Connect to the stent 410 nearby to complete the constrained path, as shown by the dashed line in FIG. 46, where one end of the pulling wire is connected to a limiting structure 4134 on the stent 410;
Method 2: Extend to the right connection hole 4135 in FIG. 46 and then follow the path of pulling wire 4602, connecting to a grid vertex or the top connection hole 4135 of the stent;
Method 3: Extend to the right connection hole 4135 in FIG. 46 and then follow the path of pulling wire 4603 to the next grid, cooperating with another pulling wire.

From the above, it can be seen that there are various flexible cooperation ways between the actuator 460, the stent 410, and the sealing assembly 420. In the embodiment shown in FIG. 47, the sealing assembly comprises a plurality of deformable sealing blocks 422 arranged at intervals, and all of the sealing blocks 422 are compressed by a single pulling wire. In this embodiment, "a single pulling wire" refers to a continuous constrained path, and does not limit the actual number of pulling wire components; for example, a single pulling wire may be formed by connecting multiple pulling components. The solution of this embodiment can be understood as the pulling wire 4603 in FIG. 46 extending circumferentially around the stent and then turning to extend axially via a connection hole 4135 or a grid vertex. Based on this embodiment, axial pulling wires may be independently provided or not provided in each grid or in multiple grids, and the cooperation between pulling wires may be achieved through threading rings or other means.

One or both ends of the pulling wire may extend out of the stent as free ends, while the remaining part of the pulling wire is threaded back and forth through the grid structure of the stent. After driving the deformation of the sealing assembly, the pulling wire may also be removed; it is not strictly required that the pulling wire be implanted into the body along with the stent during use.

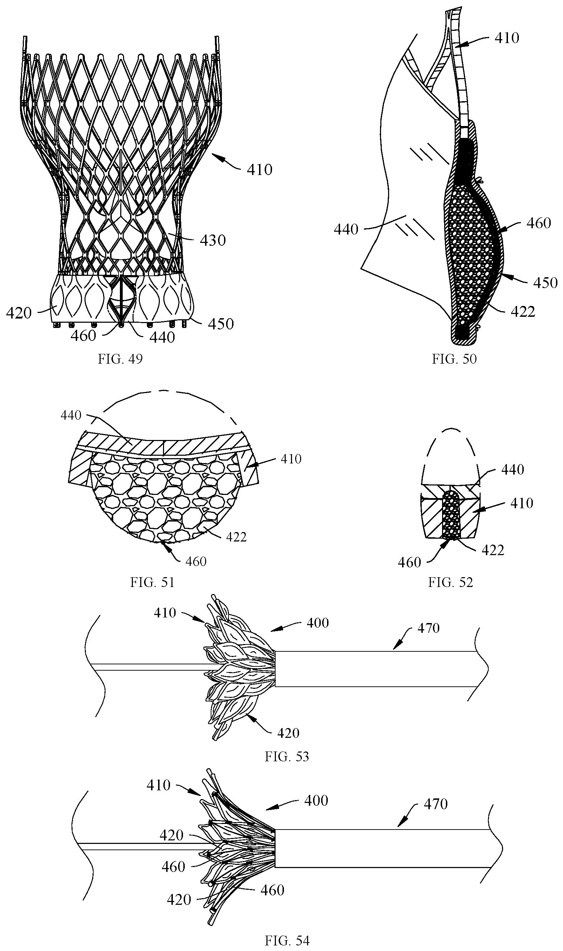

The sealing assembly and the actuator can be disposed on various stents, such as the stent 410 shown in FIGS. 47 and 48, the stent 410 shown in FIG. 49, and other types of deformable stents.

In the embodiment shown in FIGS. 49 to 52, the artificial implant further comprises:
an inner covering membrane 440 distributed around an inner circumferential wall of the stent 410, the sealing assembly 420 being fixed to an outer side of the inner covering membrane 440, and when the stent 410 is in the expanded state, the sealing assembly 420 protrudes outward of the stent 410 through corresponding grids; and
an outer covering membrane 450 distributed around an outer circumferential wall of the stent 410, wherein part or all of the sealing assembly 420 and/or the actuator 460 is wrapped by the outer covering membrane 450.

The inner covering membrane 440 and the outer covering membrane 450 may be made of PU film, and the sealing assembly 420 may be made of PU foam. PU film and PU foam have the advantages of good elasticity, easy deformation, and ability to return to their original shape. The inner and outer covering membranes can conformably adapt to the state changes of the stent 410, and the sealing assembly 420 can change its spatial volume to adapt to the state changes of the stent 410. The sealing assembly 420 may also be provided with corresponding structures, such as slits, grooves, or local reinforcements. These structures can be implemented independently to improve the conformability of the sealing assembly 420, or can optimize the cooperation between the sealing assembly and the actuator, for example, by maintaining the actuator in a specific position to improve the driving effect. Similarly, the inner and outer covering membranes may also be provided with corresponding guide structures. For example, as shown in FIGS. 51 and 52, the inner covering membrane 440 has a guide structure to improve its adaptability to state changes of the stent. The guide structure is a crease aligned with the sealing assembly 420, and may further incorporate the second guide structure and the third guide structure mentioned above.

The inflow side ends of the outer covering membrane 450 and the inner covering membrane 440 may be independently arranged, as shown in FIG. 49. Alternatively, as shown in FIG. 50, the two are connected. When they are connected and made of the same material, they may also be integrally formed, in which case the outer covering membrane 450 is understood as formed by everting the inner covering membrane 440 to the outside of the stent. Furthermore, the outer covering membrane 450 and the inner covering membrane 440 may also be implemented separately. For example, FIGS. 51 and 52 show cross-sections of the artificial implant in different states with only the inner covering membrane 440 provided.

It can be understood that an embodiment of the present application also discloses an interventional system, comprising:
an artificial implant according to any of the above technical solutions, being in a compressed state;
a catheter assembly, wherein at least one tube is inserted through the interior of the artificial implant and connected thereto; and at least one tube (e.g., the outer catheter 470 shown in FIGS. 53 and 54) wraps around the outer periphery of the artificial implant; and
a control handle connected to and driving the catheter assembly.

As shown in FIG. 53, in an embodiment without an actuator, during the loading or retrieval of the artificial implant 400 into the outer catheter 470 of the delivery system, the outer catheter 470 needs to be pushed relative to the artificial implant 400 so that the outer catheter 470 gradually wraps around the artificial implant 400. During this process, as the stent 410 is radially compressed, the sealing assembly 420 further protrudes outward from the outer periphery of the stent 410, and friction between the outer catheter 470 and the protruding sealing assembly 420 can easily cause damage to the sealing assembly 420 or even detachment from the stent 410. As shown in FIG. 54, when the stent 410 is radially compressed, the actuator 460 exerts a radially inward force on the sealing assembly 420, and the sealing assembly 420 is pressed into the interior space of the stent 410, thereby reducing the risk of damage or detachment of the sealing assembly during loading or retrieval (multiple sealing assemblies 420 and actuators 460 are shown in FIG. 54; for simplicity, only two at different positions are indicated, not all).

To load or retrieve the artificial implant, an embodiment of the present application also discloses a method for driving deformation of a sealing assembly on an artificial implant, which can be implemented through the actuators of the above embodiments. For example, the method comprises:
providing an artificial implant, the artificial implant at least comprising a stent and a sealing assembly, the sealing assembly being connected to the stent and arranged circumferentially around the stent, wherein when the stent is in the expanded state, at least a part of the sealing assembly protrudes outward from an outer periphery of the stent;
wherein a pulling wire is threaded through the stent, the pulling wire being slack to allow the sealing assembly to protrude outward, and when driving the sealing assembly to deform, the pulling wire is tightened so that it acts radially inwardly on at least a part of the sealing assembly to drive deformation of the sealing assembly. Tightening the pulling wire may be achieved entirely by deformation of the stent, or one end of the pulling wire is connected to the stent and the other end is a free end extending out of the stent, and tightening is performed by pulling the free end, or both ends of the pulling wire are free ends extending out of the stent, and tightening is performed by pulling one free end or both free ends.

For other specific embodiment processes, reference is made to the above description, which will not be repeated here.

The technical features of the above embodiments can be combined in any manner. For the sake of brevity, not all possible combinations of the technical features in the above embodiments are described. However, as long as there is no contradiction in the combination of these technical features, they should be considered within the scope of this description. When technical features from different embodiments are shown in the same drawing, that drawing shall be deemed to also disclose the combined examples of the various embodiments involved.

The above embodiments are merely several embodiments of the present application, and their descriptions are relatively specific and detailed, but they should not be construed as limiting the scope of the patent application. It should be noted that for those of ordinary skill in the art, several variations and improvements can be made without departing from the concept of the present application, and such variations and improvements shall all fall within the protection scope of the present application.

## Claims

1. An artificial implant, comprising:
a stent having a tubular structure, wherein the tubular structure is radially deformable and consequently has a corresponding compressed state and expanded state; and
a sealing assembly connected to the stent and arranged circumferentially around the stent, wherein when the stent is in the expanded state, at least a part of the sealing assembly protrudes outward from an outer periphery of the stent, and when the stent is in the compressed state, the sealing assembly is guided to fold to accommodate the deformation of the stent.

2. The artificial implant according to claim 1, wherein the deformation of the stent acts on a predetermined portion of the sealing assembly to guide the sealing assembly to fold, and during folding, at least a part of the sealing assembly moves radially inward of the stent.

3. The artificial implant according to claim 2, wherein the deformation of the stent acts directly on the sealing assembly and/or indirectly acts on the sealing assembly.

4. The artificial implant according to claim 1, wherein the sealing assembly has a first guide structure, and when the stent is in the compressed state, the sealing assembly folds at the first guide structure.

5. The artificial implant according to claim 1, further comprising:
an actuator connected to the stent, wherein during transition of the stent from the expanded state to the compressed state, the actuator acts radially inwardly on at least a part of the sealing assembly to guide folding of the sealing assembly.

6. The artificial implant according to claim 5, wherein the stent has a grid structure, and during a transition of the stent between states, an axial length of at least one grid of the grid structure changes correspondingly, and the actuator is configured to be driven by the change in axial length of the at least one grid.

7. The artificial implant according to claim 4, wherein the first guide structure comprises at least one weakened portion, and when the stent is in the compressed state, the sealing assembly folds at the at least one weakened portion, thereby being radially inwardly collapsible toward the stent.

8. The artificial implant according to claim 7, wherein the sealing assembly comprises a plurality of deformable sealing blocks arranged at intervals, and the at least one weakened portion comprises a slit formed in one of the sealing blocks.

9. The artificial implant according to claim 8, wherein a width of the slit is 0~1 mm.

10. The artificial implant according to claim 8, wherein the sealing block is made of PU foam.

11. The artificial implant according to claim 8, wherein the slit extends in a direction parallel to a generatrix of the stent.

12. The artificial implant according to claim 8, wherein in a radial direction of the stent, a depth of the slit is at least half of a thickness of the sealing block.

13. The artificial implant according to claim 8, wherein in a radial direction of the stent, a depth of the slit penetrates through the sealing block, and the sealing block is maintained connected on both sides of the slit by a first component.

14. The artificial implant according to claim 13, wherein the first component is a reinforcing wire threaded through the sealing block.

15. The artificial implant according to claim 8, wherein different parts of the sealing block have different thicknesses in a radial direction of the stent, and the slit intersects at least a part of the sealing block having the greatest thickness.

16. The artificial implant according to claim 8, wherein the slit extends axially through the sealing block, or the slit is closed on at least one side.

17. The artificial implant according to claim 8, wherein a reinforcing wire is arranged in the sealing block and is pulled on both sides of the slit.

18. The artificial implant according to claim 17, wherein a threading path of the reinforcing wire avoids the stent or wraps around the stent.

19. The artificial implant according to claim 17, wherein the reinforcing wire is fixed by tying or by an additional connector member.

20. The artificial implant according to claim 17, wherein for the same slit, the reinforcing wire is a single wire reciprocally threaded through both sides of the slit.

21. The artificial implant according to claim 17, wherein for the same slit, there are a plurality of reinforcing wires, and each reinforcing wire forms knots independently or in combination with other reinforcing wire.

22. The artificial implant according to claim 21, wherein the knots arranged at intervals along an extension direction of the slit.

23. The artificial implant according to claim 8, wherein the weakened portion is a groove formed in the sealing block.

24. The artificial implant according to claim 23, wherein an open side of the groove faces radially outward of the stent.

25. The artificial implant according to claim 23, wherein a depth of the groove is at least half of a thickness of the sealing block.

26. The artificial implant according to claim 23, wherein the groove penetrates through the sealing block, and the sealing block itself forms an annular structure.

27. The artificial implant according to claim 23, wherein the groove is located at a geometric center of the sealing block.

28. The artificial implant according to claim 4, wherein the first guide structure comprises at least one reinforced portion, and the reinforced portion drives other portions of the sealing assembly to fold and collapse radially inward of the stent.

29. The artificial implant according to claim 28, wherein the reinforced portion is formed by increasing the strength of a partial region of the sealing assembly; or the reinforced portion is formed by a second component.

30. The artificial implant according to claim 29, wherein the second component is a reinforcing wire threaded through the sealing assembly.

31. The artificial implant according to claim 28, wherein in a radial direction of the stent, a depth of the reinforced portion is part or all of a thickness of the sealing assembly.

32. The artificial implant according to claim 4, wherein the first guide structure comprises at least one weakened portion and a reinforced portion acting on the weakened portion, and the sealing assembly folds at the at least one weakened portion, thereby being radially inwardly collapsible toward the stent.

33. The artificial implant according to claim 32, wherein the weakened portion forms a cavity region, and the reinforced portion is disposed around an inner circumferential surface of the cavity region; or the reinforced portion is located on an inner circumferential surface of the cavity region close to an inner side of the stent.

34. The artificial implant according to claim 32, wherein both sides of the weakened portion are connected by a reinforcing wire.

35. The artificial implant according to claim 4, wherein the sealing assembly comprises a plurality of deformable sealing blocks, and the same sealing block has a plurality of first guide structures.

36. The artificial implant according to claim 4, wherein the sealing assembly comprises a plurality of deformable sealing blocks, and all of the sealing blocks have the first guide structure, or only some of the sealing blocks have the first guide structure.

37. The artificial implant according to claim 4, further comprising:
an actuator connected to the stent, wherein during transition of the stent from the expanded state to the compressed state, the actuator is driven by the stent to act radially inwardly on at least a part of the sealing assembly to guide folding of the sealing assembly, and the actuator is positioned in alignment with or circumferentially offset from the first guide structure.

38. The artificial implant according to claim 37, wherein the first guide structure comprises at least one weakened portion, and when the stent is in the expanded state, at least a part of the actuator extends through the weakened portion.

39. The artificial implant according to claim 1, wherein the stent is formed by cutting or braiding and has a plurality of grids, and during state switching, some or all of the grids deform adaptively.

40. The artificial implant according to claim 39, wherein the sealing assembly comprises deformable sealing blocks, and the sealing block is embedded in a correspondingly positioned grid.

41. The artificial implant according to claim 40, wherein the sealing blocks are arranged in one or more groups along an axial direction of the stent, and sealing blocks in the same group are distributed circumferentially in the same ring of grids.

42. The artificial implant according to claim 40, wherein the sealing block fills the entire grid, or only occupies a portion of the grid.

43. The artificial implant according to claim 40, wherein the sealing assembly has a first guide structure, and when the stent is in the compressed state, the sealing assembly folds at the first guide structure; and
the grid has a wide region with the largest span in a circumferential direction of the stent, and the first guide structure intersects the wide region.

44. The artificial implant according to claim 43, wherein the sealing block at least fills the wide region.

45. The artificial implant according to claim 43, wherein an extension direction of the first guide structure is perpendicular to an extension direction of the wide region.

46. The artificial implant according to claim 43, wherein in the circumferential direction of the stent, the first guide structure is located at a middle portion of the sealing block, or is offset to one side.

47. The artificial implant according to claim 43, wherein the first guide structures on all sealing blocks are offset in the same circumferential direction.

48. The artificial implant according to claim 1, further comprising:
an inner covering membrane distributed around an inner circumferential wall of the stent, the sealing assembly being fixed to an outer side of the inner covering membrane, and when the stent is in the expanded state, the sealing assembly protrudes outward of the stent through corresponding grids.

49. The artificial implant according to claim 48, wherein the stent comprises an inner frame defining a blood flow channel and an outer framework connected to the inner frame and positioned around an outer periphery of the inner frame in the expanded state, and the inner covering membrane is distributed around an inner circumferential wall of the inner frame.

50. The artificial implant according to claim 48, further comprising: when the stent is in the compressed state, a folded portion of the sealing assembly together with a corresponding portion of the inner covering membrane forms an inward protrusion relative to the inner circumferential wall of the stent.

51. The artificial implant according to claim 50, wherein in an axial view of the stent, at least 30% of the sealing assembly is located in the inward protrusion.

52. The artificial implant according to claim 48, wherein the inner covering membrane is a PU film.

53. The artificial implant according to claim 48, wherein the inner covering membrane is bonded to the sealing assembly or the sealing assembly is formed directly on a surface of the inner covering membrane.

54. The artificial implant according to claim 48, wherein an outflow side of the inner covering membrane is spliced with leaflets and fixed to the stent.

55. The artificial implant according to claim 48, wherein the inner covering membrane has a second guide structure.

56. The artificial implant according to claim 55, wherein the sealing assembly has a first guide structure, and when the stent is in the compressed state, the sealing assembly folds at the first guide structure; and
the second guide structure corresponds in position to the first guide structure in a circumferential direction of the stent.

57. The artificial implant according to claim 1, further comprising:
an outer covering membrane distributed around an outer circumferential wall of the stent, wherein part or all of the sealing assembly is wrapped by the outer covering membrane.

58. The artificial implant according to claim 57, further comprising an inner covering membrane distributed around an inner circumferential wall of the stent, wherein the outer covering membrane and the inner covering membrane are integrally formed.

59. The artificial implant according to claim 58, wherein the outer covering membrane and the inner covering membrane together define a pocket structure having a radial gap, the sealing assembly is fixed to an outer surface of the inner covering membrane, and the entire sealing assembly is located within the pocket structure, or an outflow side of the sealing assembly is exposed outside the pocket structure.

60. The artificial implant according to claim 58, wherein the inner covering membrane has a second guide structure, and the second guide structure is connected to the outer covering membrane.

61. The artificial implant according to claim 58, wherein the outer covering membrane has a third guide structure.

62. The artificial implant according to claim 61, wherein the third guide structure is connected to the inner covering membrane.

63. The artificial implant according to claim 61, wherein the sealing assembly has a first guide structure, and when the stent is in the compressed state, the sealing assembly folds at the first guide structure; and
the third guide structure is embedded in the first guide structure, and an embedded portion is fixedly connected to the sealing assembly and/or the inner covering membrane.

64. The artificial implant according to claim 61, wherein the third guide structure is fixedly connected to the sealing assembly and/or the inner covering membrane at least at an outflow side edge portion of the outer covering membrane.

65. The artificial implant according to claim 61, wherein the outer covering membrane has an axial width corresponding to at least one complete ring of grids, and the third guide structure is located at a midline portion of the corresponding grids.

66. The artificial implant according to claim 61, wherein the inner covering membrane has a second guide structure, and the outer covering membrane has a third guide structure, each of the second and third guide structures independently being a low-strength band or a pre-formed crease.

67. The artificial implant according to claim 66, wherein the low-strength band has a thinner thickness than adjacent portions or is made of a different material.

68. The artificial implant according to claim 66, wherein the pre-formed crease is obtained by reciprocally folding and then heat-treating.

69. The artificial implant according to claim 66, wherein the second guide structure and the third guide structure are respectively arranged at intervals in the circumferential direction along the stent.

70. The artificial implant according to claim 66, wherein the number of the second guide structures and the third guide structures is equal to the number of grids in an axial direction of the stent, or is an integer multiple thereof.

71. The artificial implant according to claim 1, wherein the stent has a grid structure.

72. The artificial implant according to claim 1, wherein the stent is self-expandable or balloon-expandable.

73. The artificial implant according to claim 1, wherein the stent has an inflow side and an outflow side opposite each other in an axial direction under blood flow environment, an interior of the stent defines a blood flow channel, and the artificial implant further comprises leaflets connected to the stent for controlling the blood flow channel.

74. The artificial implant according to claim 73, wherein the leaflets include 2, 3, or 4 leaflets that cooperate with each other.

75. The artificial implant according to claim 73, wherein the leaflets are made of a biological material or a polymer material.

76. The artificial implant according to claim 73, wherein the sealing assembly is located on the inflow side of the leaflets.

77. The artificial implant according to claim 1, wherein the stent comprises an inner frame defining a blood flow channel and an outer framework connected to the inner frame and positioned around an outer periphery of the inner frame in the expanded state, the outer framework being made of a memory material suitable for self-expansion.

78. The artificial implant according to claim 77, wherein the outer framework comprises arms arranged at intervals in the circumferential direction and/or an outer frame sleeved radially outside the inner frame.

79. The artificial implant according to claim 5, wherein the actuator acts on the sealing assembly in a line contact manner to radially inwardly compress the sealing assembly.

80. The artificial implant according to claim 5, wherein the actuator is made of a polymer wire, a metal wire, or a hybrid wire of polymer and metal; the sealing assembly comprises a plurality of deformable sealing blocks arranged at intervals, and the actuator compresses one of the sealing blocks or simultaneously compresses the plurality of sealing blocks.

81. The artificial implant according to claim 5, wherein the stent has a first connection point and a second connection point spaced apart in an axial direction, the actuator is a flexible member or a deformable structure, and at least one end of the actuator is connected to the first connection point, and at least the other end of the actuator is connected to the second connection point.

82. The artificial implant according to claim 5, wherein the actuator comprises at least one pulling wire, both ends of the pulling wire being constrained by two or more axially spaced portions of the stent, wherein in the expanded state the at least one pulling wire is slack to allow the sealing assembly to protrude outward, and in the compressed state the at least one pulling wire is taut to radially inwardly compress the sealing assembly.

83. The artificial implant according to claim 5, wherein the at least one pulling wire of the actuator comprises a plurality of pulling wires arranged as follows:
the plurality of pulling wires extending in an axial direction of the stent; and/or
at least one of the plurality of pulling wires extending in a circumferential direction of the stent; and/or
at least one of the plurality of pulling wires extending simultaneously in the axial and circumferential directions of the stent; and/or
a part of at least one of the plurality of pulling wires extending in the axial direction of the stent and other part extending in the circumferential direction of the stent.

84. The artificial implant according to claim 82, wherein the end of the pulling wire is connected to the stent and/or to other pulling wires by one or more of tying, threading, wrapping, bonding, and welding.

85. The artificial implant according to claim 84, wherein within at least one grid, one of the pulling wires extending in the axial direction of the stent and another one of the pulling wires extending in a circumferential direction of the stent cross each other, and the two pulling wires are positioned fixedly or movably relative to each other at a crossing point.

86. The artificial implant according to claim 84, wherein one of the pulling wires has a threading ring for other pulling wires to pass through; and the threading ring is formed by looping the pulling wire itself; or the threading ring is independently provided and connected to the corresponding pulling wire.

87. The artificial implant according to claim 84, wherein within a single grid, the pulling wires pass through a geometric center of the grid; the plurality of pulling wires cross each other, and a crossing point of the pulling wires is near the geometric center of the grid.

88. The artificial implant according to claim 84, wherein each grid provided with the sealing assembly corresponds to at least one pulling wire, and the number of pulling wires in each grid provided with the sealing assembly is the same or different.

89. The artificial implant according to claim 84, wherein the sealing assembly comprises a plurality of deformable sealing blocks arranged at intervals, and all of the sealing blocks are compressed by a single pulling wire.

90. The artificial implant according to claim 84, wherein the stent is partially hollowed to form a grid structure, and a radial dimension of the pulling wire is smaller than or close to a radial dimension of a strut of the grid structure.

91. The artificial implant according to claim 5, further comprising:
an inner covering membrane distributed around an inner circumferential wall of the stent, the sealing assembly being fixed to an outer side of the inner covering membrane, and when the stent is in the expanded state, the sealing assembly protrudes outward of the stent through corresponding grids; and
an outer covering membrane distributed around an outer circumferential wall of the stent, wherein part or all of the sealing assembly and/or the actuator is wrapped by the outer covering membrane.

92. An artificial implant, comprising:
a stent having a tubular structure and an interior defining a blood flow channel, wherein the tubular structure is radially deformable and consequently has a corresponding compressed state and expanded state;
leaflets connected to the stent for controlling the blood flow channel;
a sealing assembly connected to the stent and arranged circumferentially around the stent, wherein when the stent is in the expanded state, at least a part of the sealing assembly protrudes outward from an outer periphery of the stent; and
a pulling wire connected to the stent, wherein during a transition of the stent from the expanded state to the compressed state, the pulling wire is driven by the stent to act radially inwardly on at least a part of the sealing assembly.

93. An artificial implant, comprising:
a stent having a tubular structure, wherein the tubular structure is radially deformable and consequently has a corresponding compressed state and expanded state; and
a sealing assembly connected to the stent and arranged circumferentially around the stent, wherein when the stent is in the expanded state, at least a part of the sealing assembly protrudes outward from an outer periphery of the stent, the sealing assembly has a slit, and when the stent is in the compressed state, the sealing assembly is guided to fold at the slit to accommodate the deformation of the stent.

94. An interventional system, comprising:
an artificial implant according to any one of claims 1 to 93, being in a compressed state;
a catheter assembly, wherein at least one tube is inserted through an interior of the artificial implant and connected thereto; and at least one tube wraps around an outer periphery of the artificial implant; and
a control handle connected to and driving the catheter assembly.

95. A method for driving deformation of a sealing assembly on an artificial implant, comprising:
providing an artificial implant, the artificial implant at least comprising a stent and a sealing assembly, the sealing assembly being connected to the stent and arranged circumferentially around the stent, wherein when the stent is in an expanded state, at least a part of the sealing assembly protrudes outward from an outer periphery of the stent;
wherein a pulling wire is threaded through the stent, the pulling wire being slack to allow the sealing assembly to protrude outward, and when driving the sealing assembly to deform, the pulling wire is tightened so that it acts radially inwardly on at least a part of the sealing assembly to drive deformation of the sealing assembly.

96. A method for driving deformation of a sealing assembly on an artificial implant, comprising:
providing an artificial implant, the artificial implant at least comprising a stent and a sealing assembly, the sealing assembly being connected to the stent and arranged circumferentially around the stent, wherein when the stent is in an expanded state, at least a part of the sealing assembly protrudes outward from an outer periphery of the stent; and
the sealing assembly has a slit, and when driving the sealing assembly to deform, the sealing assembly is compressed by deformation of the stent so that it is guided to fold at the slit.
